(19) Europäisches Patentamt
European Patent Office
Office européen des brevets

(11) Veröffentlichungsnummer: **0 416 319 A2**

(12) # EUROPÄISCHE PATENTANMELDUNG

(21) Anmeldenummer: **90115376.7**

(22) Anmeldetag: **10.08.90**

(51) Int. Cl.[5]: **C07D 239/52**, C07D 239/47, C07D 239/42, C07D 403/12

(30) Priorität: **23.08.89 DE 3927788**

(43) Veröffentlichungstag der Anmeldung: **13.03.91 Patentblatt 91/11**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Lantzsch, Reinhard, Dr.**
**Am Buschhäuschen 51**
**W-5600 Wuppertal 1(DE)**
Erfinder: **Müller, Klaus-Helmut, Dr.**
**Bockhackstrasse 55**
**W-4000 Düsseldorf 13(DE)**
Erfinder: **Littmann, Martin, Dr.**
**Hahnenweg 1**
**W-5000 Köln 80(DE)**

(54) **Verfahren zur Herstellung von Sulfonylisoharnstoffen.**

(57) Die bekannten, herbizid wirksamen Sulfonylisoharnstoffe der Formel (I),

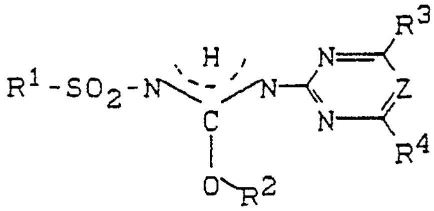

(I)

(worin Z für CH oder N steht und die Reste $R^1$, $R^2$, $R^3$ und $R^4$ die in der Beschreibung genannten Bedeutungen haben), erhält man in guten Ausbeuten und hoher Reinheit durch Umsetzung von Isoharnstoffen der Formel (II)

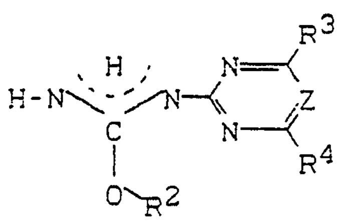

(II)

mit Sulfonsäurehalogeniden der Formel (III)
$R^1$-$SO_2$-X (III)
(worin X für Halogen steht) in Gegenwart eines Saureakzeptors und gegebenenfalls in Gegenwart eines Verdünnungsmittels bei Temperaturen zwischen -20 °C und +50 °C.

Die Zwischenprodukte der Formel (II) können nach einem gleichfalls erfinderischen Verfahren hergestellt werden, indem man Isoharnstoffe der Formel (IV) oder deren Säureaddukte

EP 0 416 319 A2

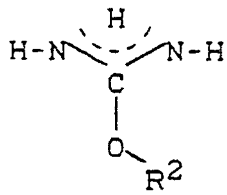

(IV)

mit Azinen der Formel (V)

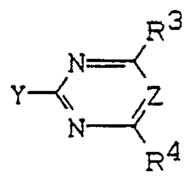

(V)

(worin Y für Halogen oder Alkylsulfonyl steht), in Gegenwart eines Säureakzeptors und in Gegenwart eines Verdünnungsmittels bei 0°-200°C umsetzt.

Eine bestimmte, definierte Untergruppe von Isoharnstoffen der Formel (II) - in der Beschreibung mit (IIa) bezeichnet - ist neu und ist ebenfalls Gegenstand der Erfindung.

# VERFAHREN ZUR HERSTELLUNG VON SULFONYLISOHARNSTOFFEN

Die Erfindung betrifft ein neues Verfahren und neue Zwischenprodukte zur Herstellung von bekannten herbizid wirksamen Sulfonylisoharnstoffen, ferner ein neues Verfahren zur Herstellung der Zwischenprodukte (II).

Es ist bekannt, daß man bestimmte Sulfonylisoharnstoffe, wie z.B. N′-(4-Methoxy-6-methyl-s-triazin-2-yl)-N″-(2-chlor-benzolsulfonyl)-O-methyl-isoharnstoff, erhält, wenn man entsprechende Sulfonylharnstoffe, wie z.B. N′-(4-Methoxy-6-methyl-s-triazin-2-yl)-N″-(2-chlor-benzolsulfonyl)-harnstoff, mit Triphenylphosphin und Tetrachlormethan und - ohne Zwischenisolierung der hierbei gebildeten N-Sulfonyl-imino-carbamidsäure-chloride -weiter mit Alkoholen, wie z.B. Methanol, oder Alkoholaten, wie z.B. Natriummethanolat, umsetzt (vergl. EP-A 24 215). Die Sulfonylisoharnstoffe fallen hierbei jedoch stark verunreinigt an und bedürfen der Reinigung, beispielsweise durch Säulenchromatographie.

Weiter ist bekannt, daß man bestimmte Sulfonylisoharnstoffe, wie z.B. N-(2-Chlor-benzolsulfonyl)-imino-N′-(4′-methoxy-6′-methyl-triazinyl)-O-methyl-isoharnstoff, erhält, wenn man entsprechende N-Sulfonyliminothiokohlensäureester, wie z.B. N-(2-Chlor-benzolsulfonyl)iminothiokohlensäure-O,S-dimethylester, mit geeigneten Aminoverbindungen, wie z.B. 2-Amino-4-methoxy-6-methyl-triazin umsetzt (vgl. CH-P 646957). Abgesehen davon, daß bei diesem Verfahren übelriechendes Mercaptan freigesetzt wird, sind auch die Ausbeuten nicht zufriedenstellend.

Ferner ist bekannt, daß man bestimmte Sulfonylisoharnstoffe, wie z.B. N′-(4,6-Dimethyl-pyrimidin-2-yl)-N″-(2-chlor-benzolsulfonyl)-O-(1-methylethyl)-isoharnstoff, erhält, wenn man geeignete Sulfonylguanidine, wie z.B. N′-(4,6-Dimethyl-pyrimidin-2-yl)-N″-methoxy-N″,N‴-bis-(2-chlor-benzolsulfonyl)-guanidin, mit Alkoholen, wie z.B. Isopropanol, umsetzt (vgl. EP-A 173 311). Die Herstellung der hierbei als Ausgangsstoffe einzusetzenden Sulfonylguanidine verläuft jedoch über einen vielstufigen Syntheseweg in oft nicht befriedigenden Ausbeuten.

Es wurde nun gefunden, daß man Sulfonylisoharnstoffe der allgemeinen Formel (I)

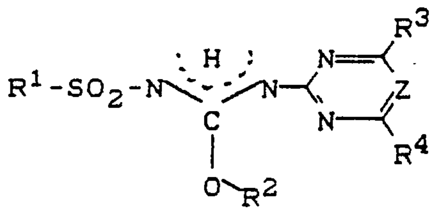

(I)

in welcher

$R^1$ für einen gegebenenfalls substituierten Rest aus der Reihe Alkyl, Aralkyl, Aryl oder Heteroaryl steht,

$R^2$ für Alkyl oder Aralkyl steht,

$R^3$ für Wasserstoff, Halogen oder für jeweils gegebenenfalls substituiertes Alkyl oder Alkoxy steht,

$R^4$ für Wasserstoff, Halogen oder für jeweils gegebenenfalls substituiertes Alkyl oder Alkoxy steht und

Z für Stickstoff oder eine CH-Gruppierung steht,

in guten Ausbeuten und in hoher Reinheit erhält, wenn man Isoharnstoffe der allgemeinen Formel (II)

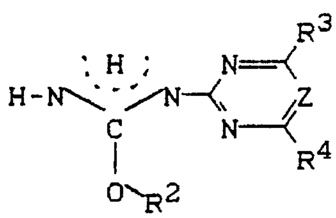

(II)

in welcher

$R^2$, $R^3$, $R^4$ und Z die oben angegebenen Bedeutungen haben,

mit Sulfonsäurehalogeniden der allgemeinen Formel (III)

$R^1$-$SO_2$-X (III)

in welcher

$R^1$ die oben angegebene Bedeutung hat und

X für Halogen steht,
in Gegenwart eines Säureakzeptors und gegebenenfalls in Gegenwart eines Verdünnungsmittels bei Temperaturen zwischen -20 °C und +50 °C umsetzt.

Es ist als überraschend anzusehen, daß man nach dem erfindungsgemäßen Verfahren aus Isoharnstoffen und Sulfonsäurehalogeniden die Sulfonylisoharnstoffe der Formel (I) in guten Ausbeuten und in hoher Reinheit erhält, da bekannt war, daß beispielsweise strukturell ähnliche Iminoether (Imidoester) mit Sulfonsäurechloriden Entalkylierungsprodukte bilden (vgl. J. Chem. Soc. 1943, 101-104).

Vorteile des erfindungsgemäßen Verfahrens liegen beispielsweise darin, daß die benötigten Ausgangsstoffe in wenigen Syntheseschritten, unter Verwendung preiswerter Chemikalien in guten Ausbeuten erhalten werden können.

Verwendet man beispielsweise 2-Fluor-benzolsulfonsäurechlorid und N-(4,6-Dimethyl-pyrimidin-2-yl)-O-methylisoharnstoff als Ausgangsstoffe, so kann der Reaktionsablauf durch das folgende Formelschema skizziert werden:

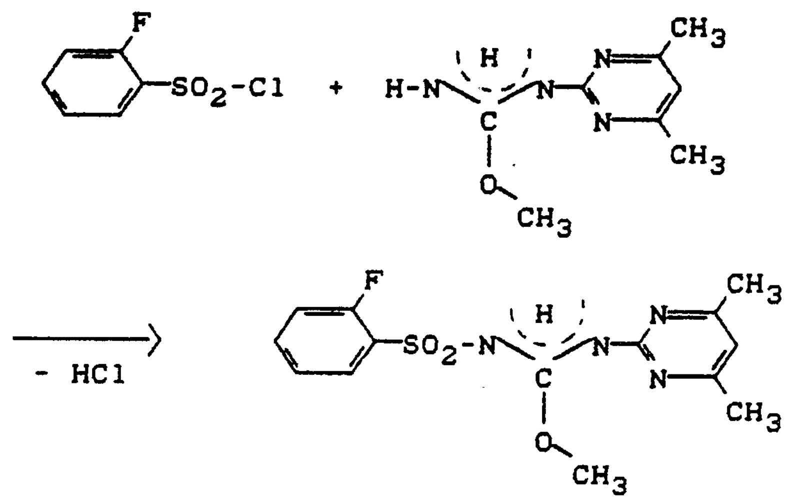


Die als Ausgangsstoffe zu verwendenden Isoharnstoffe sind durch die Formel (II) allgemein definiert. In Formel (II) stehen vorzugsweise
$R^2$ für $C_1$-$C_4$-Alkyl oder für Benzyl,
$R^3$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy oder $C_1$-$C_2$-Alkoxy-$C_1$-$C_2$-alkoxy,
$R^4$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$- $C_4$-Alkoxy, $C_1$-$C_4$-Halogenalkoxy, oder $C_1$-$C_2$-Alkoxy-$C_1$-$C_2$-alkoxy und
Z für Stickstoff oder eine CH-Gruppierung.

Als Ausgangsstoffe besonders bevorzugt werden die Verbindungen der Formel (II), in welcher
$R^2$ für Methyl oder Ethyl steht,
$R^3$ für Chlor, Methyl, Ethyl, Trifluormethyl, Methoxymethyl, Ethoxymethyl, Methoxy, Ethoxy oder Difluormethoxy steht,
$R^4$ für Chlor, Methyl, Ethyl, Trifluormethyl, Methoxy, Ethoxy oder Difluormethoxy steht, und
Z für eine CH-Gruppierung steht.

Als Beispiele für die Ausgangsstoffe der Formel (II) seien genannt:
N-(4,6-Dichlor-pyrimidin-2-yl)-, N-(4,6-Dimethyl-pyrimidin-2-yl)-, N-(4-Chlor-6-methyl-pyrimidin-2-yl)-, N-(4,6-Dimethoxy-pyrimidin-2-yl)-, N-(4,6-Diethoxy-pyrimidin-2-yl)-, N-(4-Methoxy-6-methyl-pyrimidin-2-yl)-, N-(4-Ethoxy-6-methyl-pyrimidin-2-yl)-, N-(4-Ethyl-6-methoxy-pyrimidin-2-yl)-, N-(4-Methoxy-6-trifluormethyl-pyrimidin-2-yl)-, N-(4,6-Bis-trifluormethyl-pyrimidin-2-yl)- und N-(4,6-Bis-difluormethoxy-pyrimidin-2-yl)-O-methyl-isoharnstoff.

Die Isoharnstoffe der Formel (II) sind mit Ausnahme von einigen N-(4,6-Dimethyl-pyrimidin-2-yl)-O-alkyl-isoharnstoffen noch nicht aus der Literatur bekannt (vgl. Chem. Pharm. Bull. 21 (1973), 478-482).

Neu und ebenfalls Gegenstand der vorliegenden Erfindung sind die Isoharnstoffe der Formel (IIa),

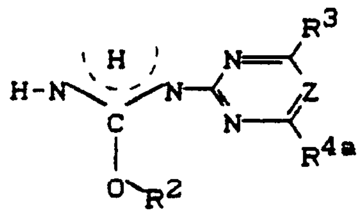

(IIa)

in welcher
$R^2$ für Alkyl oder Aralkyl steht,
$R^3$ für Wasserstoff, Halogen oder für jeweils gegebenenfalls substituiertes Alkyl oder Alkoxy steht,
$R^{4a}$ für Wasserstoff, Halogen oder für gegebenenfalls substituiertes Alkoxy steht und
Z für Stickstoff oder eine CH-Gruppierung steht.

Bevorzugte neue Isoharnstoffe sind Verbindungen der Formel (IIa),
in welcher
$R^2$ für $C_1$-$C_4$-Alkyl oder für Benzyl steht,
$R^3$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy oder $C_1$-$C_2$-Alkoxy-$C_1$-$C_2$-alkoxy steht,
$R^{4a}$ für Wasserstoff, Fluor, Chlor, Brom, $C_1$-$C_4$-Alkoxy, $C_1$-$C_4$-Halogenalkoxy oder $C_1$-$C_2$-Alkoxy-$C_1$-$C_2$-alkoxy steht und
Z für Stickstoff oder eine CH-Gruppierung steht.

Besonders bevorzugte neue Isoharnstoffe sind Verbindungen der Formel (IIa),
in welcher
$R^2$ für Methyl oder Ethyl steht,
$R^3$ für Chlor, Methyl, Ethyl, Trifluormethyl, Methoxymethyl, Ethoxymethyl, Methoxy, Ethoxy oder Difluormethoxy steht,
$R^{4a}$ für Chlor, Methoxy, Ethoxy oder Difluormethoxy steht und
Z für eine CH-Gruppierung steht.

Man erhält die Verbindungen der Formel (II) bzw. (IIa) nach einem neuen und erfinderischen Verfahren, wenn man Isoharnstoffe der allgemeinen Formel (IV)

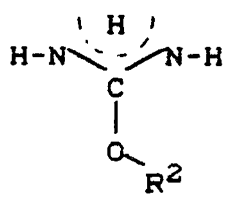

(IV)

in welcher
$R^2$ die oben angegebene Bedeutung hat,
- oder Säureaddukte von Verbindungen der Formel (IV) -
mit Azinen der allgemeinen Formel (V)

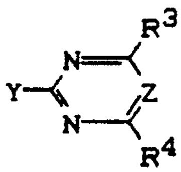

(V)

in welcher
$R^3$ und $R^4$ die oben angegebenen Bedeutungen haben,
Y für Halogen oder Alkylsulfonyl steht, und
Z für Stickstoff oder eine CH-Gruppierung steht,
in Gegenwart eines Säureakzeptors, wie z.B. Kaliumcarbonat, und in Gegenwart eines Verdünnungsmittels, wie z.B. Aceton, Acetonitril oder Dimethylformamid, bei Temperaturen zwischen 0 °C und 200 °C, vorzugsweise zwischen 10 °C und 150 °C, umsetzt (vgl. Herstellungsbeispiele).

Die bekannten N-(4,6-Dimethyl-pyrimidin-2-yl)-O-alkylisoharnstoffe werden nach der Literatur (vgl. Chem. Pharm. Bull. 21 (1973), 478-482) durch Umsetzung von N-Amidino-O-alkyl-isoharnstoffen mit Acetylaceton erhalten. Diese Synthesemethodik ist jedoch nur in beschränktem Umfang zur Herstellung von Verbindungen der Formel (II) anwendbar. Das erfindungsgemäße Verfahren zur Herstellung von Verbindungen der Formel (II) aus Isoharnstoffen der Formel (IV) und Azinen der Formel (V) ermöglicht demgegenüber die Herstellung einer Vielzahl von neuen, sonst nicht zugänglichen Verbindungen der Formel (II) auf einfache Weise und in überraschend hohen Ausbeuten. Als besonders überraschend ist anzusehen, daß auch bei der Umsetzung von mehrfach halogenierten Azinen, wie z.B. von 2,4,6-Trichlor-pyrimidin, mit Isoharnstoffen der Formel (IV) die Substitutionsreaktion praktisch nur in 2-Position unter Bildung von Verbindungen der Formel (II) efolgt, da nach der Literatur eine gleichmäßige Verteilung der möglichen Substitutionsprodukte zu erwarten war (vgl. D.J. Brown, The Pyrimidines, S.188-189, Interscience Publishers, John Wiley & Sons, New York-London, 1962).

Die zweistufige Reaktionsfolge - zunächst die Umsetzung von Isoharnstoffen der Formel (IV) mit Azinen der Formel (V) und anschließende Umsetzung der dadurch erhaltenen Isoharnstoffe der Formel (II) mit Sulfonsäurehalogeniden der Formel (III) - ist ebenfalls Gegenstand der Erfindung.

Die als Vorprodukte benötigten Isoharnstoffe sind durch die Formel (IV) allgemein definiert. In Formel (IV) hat $R^2$ vorzugsweise bzw. insbesondere diejenige Bedeutung, die bereits im Rahmen der Beschreibung der Ausgangsstoffe der Formel (II) als bevorzugt bzw. als insbesondere bevorzugt für $R^2$ angegeben wurden.

Als Beispiele für die Ausgangsstoffe der Formel (IV) seien genannt:
O-Methyl-isoharnstoff und O-Ethyl-isoharnstoff.

Bevorzugte Säureaddukte der Verbindungen der Formel (IV) sind ihre Hydrogensulfate oder ihre Sulfate.

Die Isoharnstoffe der Formel (IV) bzw. ihre Säureaddukte sind bekannte organische Synthesechemikalien.

Die weiter als Vorprodukte benötigten Azine sind durch die Formel (V) allgemein definiert. In Formel (V) haben $R^3$ und $R^4$ vorzugsweise bzw. insbesondere diejenigen Bedeutungen, die bereits im Rahmen der Beschreibung der Ausgangsstoffe der Formel (II) als bevorzugt bzw. als insbesondere bevorzugt für $R^3$ und $R^4$ angegeben wurden. Y steht vorzugsweise für Chlor oder Methylsulfonyl und Z für Stickstoff oder eine CH-Gruppierung.

Als Beispiele für die Ausgangsstoffe der Formel (V) seien genannt:
2-Chlor- und 2-Methylsulfonyl-4,6-dichlor-pyrimidin, -4-chlor-6-methyl-pyrimidin, -4-chlor-6-methoxy-pyrimidin, -4,6-dimethoxy-pyrimidin, -4,6-diethoxy-pyrimidin, -4-methoxy-6-methyl-pyrimidin, -4-ethoxy-6-methyl-pyrimidin, -4-ethyl-6-methoxy-pyrimidin, -4-methoxy-6-trifluormethyl-pyrimidin, -4,6-bis-trifluormethyl-pyrimidin, -4,6-bis-difluormethoxy-pyrimidin und -4-methyl-6-difluormethoxy-pyrimidin.

Die Azine der Formel (V) sind bekannt und/oder können nach an sich bekannten Verfahren hergestellt werden (vgl. J. Chem Soc. 1957, 1830-1833; J. Org. Chem. 26 (1961), 792; US-P 3 308 119 und US-P 4 711 959).

Die Ausgangsstoffe der Formel (II), in welcher $R^3$ und $R^4$ für Alkoxy stehen, werden vorzugsweise in einer Synthesefolge erhalten, bei der man 2,4,6-Trichlor-pyrimidin mit einem Isoharnstoff der Formel (IV) - oben -oder einem Säureaddukt hiervon in Gegenwart eines Säureakzeptors, wie z.B. Natriumcarbonat, Kaliumcarbonat, Calciumcarbonat, Triethylamin, Pyridin, N,N-Dimethylbenzylamin oder N,N-Dimethyl-cyclohexylamin, und in Gegenwart eines Verdünnungsmittels, wie z.B. Toluol, Xylol, Methylcyclohexan, Diisopropylether, Tetrahydrofuran, Dioxan, 1,2-Dimethoxy-ethan, Methyl-tert-butylether oder Methyl-tert-amyl-ether, bei Temperaturen zwischen 0 °C und 200 °C, vorzugsweise zwischen 70 °C und 150 °C, umsetzt und die hierbei erhaltenen Verbindungen der Formel (II), in welcher $R^3$ und $R^4$ für Chlor stehen, mit Alkalimetallalkanolaten in Gegenwart der entsprechenden Alkohole bei Temperaturen zwischen 0 °C und 150 °C, vorzugsweise zwischen 10 °C und 100 °C umsetzt (vgl. Beispiel (II-2/II-3)).

Überraschenderweise gelingt die Umsetzung von N-(4,6-Dichlor-pyrimidin-2-yl)-O-alkyl-isoharnstoffen mit Alkalimetallalkoholaten zu N-(4,6-Dialkoxy-pyrimidin-2-yl)-O-alkyl-isoharnstoffen praktisch quantitativ bei mäßig erhöhten Temperaturen (beispielsweise bei Methanol-Rückflußtemperatur), während beispielsweise die Umsetzung von 2-Amino-4,6-dichlor-pyrimidin mit Natriummethylat zu 4-Amino-4,6-dimethoxy-pyrimidin bei 140 °C bis 150 °C durchzuführen ist (vgl. J. Chem Soc. 1946, 81-85).

Die beim erfindungsgemäßen Verfahren zur Herstellung von Sulfonylisoharnstoffen der Formel (I) weiter als Ausgangsstoffe zu verwendenden Sulfonsäurehalogenide sind durch die Formel (III) allgemein definiert. In Formel (III) steht vorzugsweise
$R^1$ für den Rest

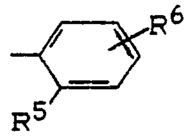

,

worin

$R^5$ für Wasserstoff, Halogen, Cyano, Nitro, $C_1$-$C_4$-Alkyl (welches gegebenenfalls durch Halogen, Cyano, $C_1$-$C_4$-Alkoxy oder $C_1$-$C_4$-Alkoxy-carbonyl substituiert ist), $C_1$-$C_4$-Alkoxy (welches gegebenenfalls durch Halogen, Cyano, $C_1$-$C_4$-Alkoxy oder $C_1$-$C_4$-Alkoxy-carbonyl substituiert ist), $C_1$-$C_4$-Alkylthio, $C_1$-$C_4$-Alkylsulfinyl oder $C_1$-$C_4$-Alkylsulfonyl (welche gegebenenfalls durch Halogen, Cyano, $C_1$-$C_4$-Alkoxy oder $C_1$-$C_4$-Alkoxy-carbonyl substituiert sind), für Di-($C_1$-$C_4$-alkyl)-aminosulfonyl, N-($C_1$-$C_4$-Alkoxy)-N-$C_1$-$C_4$-alkyl-aminosulfonyl, Phenyl, Phenoxy, $C_1$-$C_4$-Alkoxy-carbonyl oder Di-($C_1$-$C_4$-alkylamino)-carbonyl steht und

$R^6$ für Wasserstoff oder Halogen steht,

oder es steht vorzugsweise

$R^1$ für den Rest

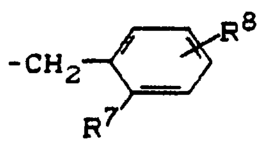

,

worin

$R^7$ für Wasserstoff, Halogen, Cyano, Nitro, $C_1$-$C_4$-Alkyl (welches gegebenenfalls durch Halogen, Cyano, $C_1$-$C_4$-Alkoxy oder $C_1$-$C_4$-Alkoxy-carbonyl substituiert ist), $C_1$-$C_4$-Alkoxy (welches gegebenenfalls durch Halogen, Cyano, $C_1$-$C_4$-Alkoxy oder $C_1$-$C_4$-Alkoxy-carbonyl substituiert ist), $C_1$-$C_4$-Alkylthio, $C_1$-$C_4$-Alkylsulfinyl oder $C_1$-$C_4$-Alkylsulfonyl (welche gegebenenfalls durch Halogen, Cyano, $C_1$-$C_4$-Alkoxy oder $C_1$-$C_4$-Alkoxy-carbonyl substituiert sind), für Di-($C_1$-$C_4$-alkyl)-aminosulfonyl oder $C_1$-$C_4$-Alkoxy-carbonyl steht und

$R^8$ für Wasserstoff oder Halogen steht,

oder es steht vorzugsweise

$R^1$ für den Rest

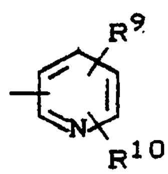

,

worin

$R^9$ für Wasserstoff, Halogen, Cyano, Nitro, $C_1$-$C_4$-Alkyl (welches gegebenenfalls durch Halogen substituiert ist), $C_2$-$C_4$-Alkenyl (welches gegebenenfalls durch Halogen substituiert ist), $C_1$-$C_4$-Alkoxy (welches gegebenenfalls durch Halogen substituiert ist), $C_1$-$C_4$-Alkylthio, $C_1$-$C_4$-Alkylsulfinyl oder $C_1$-$C_4$-Alkylsulfonyl (welche gegebenenfalls durch Halogen substituiert sind), Di-($C_1$-$C_4$-alkyl)-aminosulfonyl, $C_1$-$C_4$-Alkoxycarbonyl, Di-($C_1$-$C_4$-alkyl)-aminocarbonyl oder Dioxolanyl steht und

$R^{10}$ für Wasserstoff oder Halogen steht,

oder es steht vorzugsweise

$R^1$ für den Rest

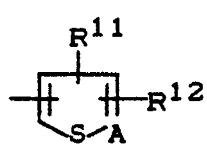

,

worin

A für Stickstoff oder eine CH-Gruppierung steht,

$R^{11}$ für Wasserstoff, Halogen, Cyano, Nitro, $C_1$-$C_4$-Alkyl (welches gegebenenfalls durch Halogen, $C_1$-$C_4$-

Alkoxy oder $C_1$-$C_4$-Halogenalkoxy substituiert ist), $C_1$-$C_4$-Alkoxy (welches gegebenenfalls durch Halogen oder $C_1$-$C_4$-Alkoxy substituiert ist), $C_1$-$C_4$-Alkylthio, $C_1$-$C_4$-Alkylsulfinyl oder $C_1$-$C_4$-Alkylsulfonyl (welche gegebenenfalls durch Halogen substituiert sind), Di-($C_1$-$C_4$-alkyl)-aminosulfonyl oder $C_1$-$C_4$-Alkoxycarbonyl steht und
$R^{12}$ für Wasserstoff oder Halogen steht,
oder es steht vorzusweise
$R^1$ für den Rest

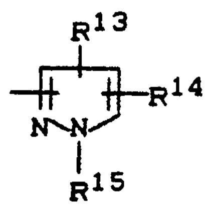

,

worin
$R^{13}$ für Wasserstoff, Halogen, Cyano, Nitro, $C_1$-$C_4$-Alkyl (welches gegebenenfalls durch Halogen substituiert ist), $C_1$-$C_4$-Alkoxy (welches gege benenfalls durch Halogen substituiert ist), Dioxolanyl cder $C_1$-$C_4$-Alkoxycarbonyl steht,
$R^{14}$ für Wasserstoff oder Halogen steht und
$R^{15}$ für Wasserstoff, $C_1$-$C_4$-Alkyl, Phenyl oder (Iso)Chinolinyl steht,
oder es steht vorzugsweise
$R^1$ für den Rest

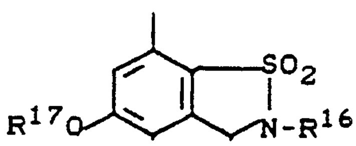

,

worin
$R^{16}$ und $R^{17}$ für $C_1$-$C_4$-Alkyl stehen,
oder es steht vorzugsweise
$R^1$ für den Rest

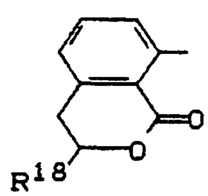

,

worin
$R^{18}$ für Wasserstoff oder Methyl steht, und
X steht vorzugsweise für Fluor, Chlor oder Brom.

Als Ausgangsstoffe besonders bevorzugt werden die Verbindungen der Formel (III), in welcher
$R^1$ für den Rest

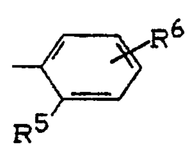

steht, worin
$R^5$ für Fluor, Chlor, Brom, Methyl, Trifluormethyl, Methoxy, Difluormethoxy, Trifluormethoxy, Methylthio,

Methylsulfonyl, Dimethylaminosulfonyl, N-Methoxy-N-methyl-aminosulfonyl, Phenyl, Methoxycarbonyl oder Ethoxycarbonyl steht und

$R^6$ für Wasserstoff, Fluor oder Chlor steht, oder

$R^1$ für den Rest

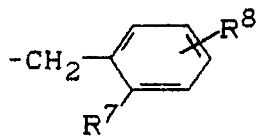

steht, worin

$R^7$ für Fluor, Chlor, Brom, Methyl, Methoxy, Ethoxy, Difluormethoxy, Trifluormethoxy, Methoxycarbonyl oder Ethoxycarbonyl steht und

$R^8$ für Wasserstoff, oder

$R^1$ für den Rest

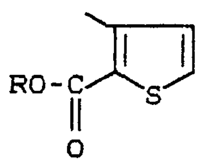

steht, worin

R für Methyl oder Ethyl steht, oder

$R^1$ für den Rest

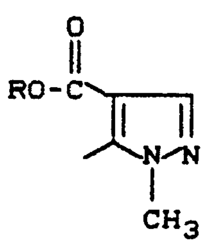

steht, worin

R für Methyl oder Ethyl steht,

und in welcher weiter

X für Chlor steht.

Als Ausgangsstoffe der Formel (III) seien beispielsweise genannt:

2-Chlor-, 2,6-Dichlor-, 2,5-Dichlor-, 2-Fluor-, 2-Brom-, 2-Methyl-, 2-Trifluormethyl-, 2-Methoxy-, 2-(2-Methoxyethoxy)-, 2-Methylsulfonyl-, 2-Chlor-6-methyl-, 2-Brom-6-methyl-, 2-Methylthio-, 2-Trifluormethylthio-, 2-Difluormethylthio-, 2-Phenoxy-, 2-Difluormethoxy-, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonyl-benzolsulfonsäurechlorid sowie (2-Chlor-phenyl)-, (2-Cyano-phenyl)-, (2-Methoxycarbonylphenyl)-, (2-Trifluormethoxy-phenyl)- und (2-Difluormethoxy-phenyl)-methansulfonsäurechlorid, ferner 1-Methyl-4-methoxycarbonyl-pyrazol-5-sulfonsäurechlorid, 1-Methyl-4-ethoxycarbonyl-pyrazol-5-sulfonsäurechlorid, 1-Methyl-4-brom-pyrazol-5-sulfonsäurechlorid, 2-Methoxycarbonyl-thiophen-3-sulfonsäurechlorid, 3-Trifluormethyl-pyridin-2-sulfonsäurechlorid, 3-Dimethylaminocarbonyl-pyridin-2-sulfonsäurechlorid, 3-Dimethylaminocarbonyl-6-methyl-pyridin-2-sulfonsäurechlorid, 3-Dimethylaminocarbonyl-6-chlor-pyridin-2-sulfonsäurechlorid und 1-(Iso)Chinolinyl-4-ethoxycarbonyl-pyrazol-5-sulfonsäurechlorid

Die Sulfonsäurehalogenide der Formel (III) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Das erfindungsgemäße Verfahren zur Herstellung von Sulfonylisoharnstoffen der Formel (I) wird in Gegenwart eines Säureakzeptors durchgeführt.

Als Säureakzeptoren können bei dem erfindungsgemäßen Verfahren alle üblicherweise für derartige Umsetzungen verwendbaren Säurebindemittel eingesetzt werden Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliumtert-butylat, ferner aliphatische, aromatische oder heterocyclische Amine, beispielsweise Dicyclohexylamin, Diisopropylamin, Triethylamin, Trimethylamin, Tributylamin, Dimethylanilin, Dimethylbenzylamin, Dimethylcyclohexylamin, Pyridin, alkylierte Pyridine, wie 5-Ethyl-2-methyl-pyridin, Picoline, Lutidine und Collidine, ferner 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die als Säureakzeptoren genannten Amine, insbesondere Dicyclohexylamin, werden als Säurebindemittel besonders bevorzugt.

Das erfindungsgemäße Verfahren zur Herstellung der Sulfonylharnstoffe der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Methylcyclohexan, Petrolether, Benzin, Ligroin, Dekalin, Benzol, Toluol, Xylol, Methylenchlorid, Ethylenchlorid, Chloroform, Tetrachlorkohlenstoff, Chlorbenzol und o-Dichlorbenzol, Ether wie Diethyl-, Diisopropyl- und Dibutylether, Methyl-tert-butylether, Methyl-tert-amylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid, ferner auch die oben angegebenen Amine, sofern sie als Säureakzeptoren eingesetzt werden.

Bevorzugte Verdünnungsmittel für das erfindungsgemäße Verfahren sind die oben angegebenen Kohlenwasserstoffe und Ether.

Die Reaktionstemperaturen können bei dem erfindungsgemäßen Verfahren in einem größeren Bereich variiert werden. Im allgemeinen arbeitet man bei Temperaturen zwischen -20 °C und +50 °C, vorzugsweise bei Temperaturen zwischen 0 °C und 30 °C.

Das erfindungsgemäße Verfahren wird im allgemeinen unter Normaldruck durchgeführt. Es ist jedoch auch möglich, unter erhöhtem oder vermindertem Druck (im allgemeinen zwischen 0,1 und 10 bar) zu arbeiten.

Zur Durchführung des erfindungsgemäßen Verfahrens setzt man auf 1 Mol Isoharnstoff der Formel (II) im allgemeinen zwischen 0,9 und 1,5 Mol, vorzugsweise zwischen 1,0 und 1,2 Mol, Sulfonsäurehalogenid der Formel (III) und zwischen 1 und 2 Mol, vorzugsweise zwischen 1,0 und 1,2 Mol, eines Säureakzeptors ein.

Zur Durchführung des erfindungsgemäßen Verfahrens können die Reaktioneskomponenten in beliebiger Reihenfolge vermischt und nach beendeter Umsetzung nach üblichen Methoden aufgearbeitet werden.

In einer bevorzugten Ausführungsform des erfindungsgemäßen Verfahrens wird der Isoharnstoff der Formel (II) mit einem Säureakzeptor und gegebenenfalls einem Verdünnungsmittel verrührt und das Sulfonsäurehalogenid der Formel (III) wird dann unter leichtem Kühlen langsam dazugegeben. Das Reaktionsgemisch wird dann bis zum Ende der Umsetzung gerührt.

Die Aufarbeitung kann beispielsweise wie folgt durchgeführt werden:

Man engt das Reaktionsgemisch ein, verrührt den Rückstand mit Wasser und isoliert das kristalline Produkt durch Absaugen. Die weitere Reinigung des Produktes kann durch Waschen mit einem organischen Lösungsmittel, wie z.B. Toluol, oder durch Umkristallisation erfolgen.

Die nach dem erfindungsgemäßen Verfahren herstellbaren Sulfonylisoharnstoffe der Formel (I) können als Herbizide verwendet werden (vgl. CH-P 646 957, EP-A 24215, DE-OS 36 18 004).

Herstellungsbeispiele

Beispiel 1

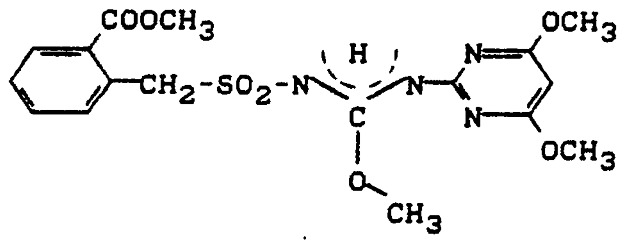

21,2 g (0,1 Mol) N-(4,6-Dimethoxy-pyrimidin-2-yl)-O-methyl-isoharnstoff und 19,9 g (0,11 Mol) Dicyclohexylamin werden in 100 ml Dimethoxyethan vorgelegt und bei 5 bis 10 ° C werden 29,8 g (ca. 90%ig, 0,108 Mol) in 60 ml Dimethoxyethan gelöstes 2-Methoxycarbonyl-benzylsulfonsäurechlorid zugetropft. Man läßt auf Raumtemperatur kommen und rührt 12 Stunden nach. Nach Einengen am Rotationsverdampfer wird der Rückstand in Wasser aufgenommen, 15 Minuten verrührt und abgesaugt. Nach Ausrühren mit Toluol und erneutem Absaugen erhält man 36,3 g weiß-kristallines Produkt, welches 95 % N-(4,6-Dimethoxy-pyrimidin-2-yl)-N'-(2-methoxycarbonyl-benzylsulfonyl)-O-methyl-isoharnstoff enthält.
Ausbeute: 82 % der Theorie; Schmelzpunkt: 124-126 ° C

Ausgangsstoffe der Formel (II)

Beispiel (II-1)

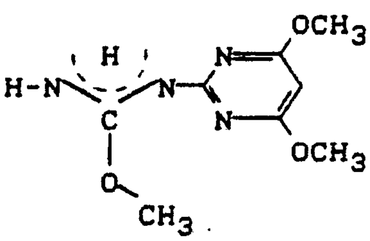

54,5 g (0,25 Mol) 2-Methylsulfonyl-4,6-dimethoxy-pyrimidin, 69 g (0,5 Mol) Kaliumcarbonat und 1500 ml Acetonitril werden unter Rühren vermischt. Dann gibt man 33,85 g (0,1375 Mol) O-Methyl-isoharnstoffsulfat zu und erhitzt 17 Stunden zum Sieden. Nach Abkühlen wird abgesaugt und die Mutterlauge eingeengt. Nach Aufnehmen in Methylenchlorid wäscht man dreimal mit Wasser, trocknet und engt erneut ein.
Man erhält 49,9 g N-(4,6-Dimethoxy-pyrimidin-2-yl)-O-methyl-isoharnstoff in Form gelber Kristalle vom Schmelzpunkt 91 - 95 ° C (Gehalt 90 %; Ausbeute: 85 % der Theorie).

Beispiel (II-2)

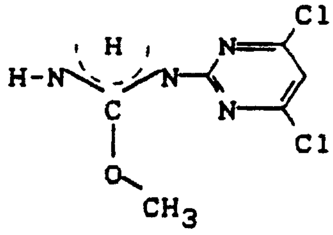

45,9 g (0,25 Mol) 2,4,6-Trichlorpyrimidin, 30,8 g (0,125 Mol) O-Methyl-isoharnstoffsulfat und 51,8 g (0,375 Mol) gemahlenes Kaliumcarbonat werden in 800 ml Toluol suspendiert und 10 Stunden bei 110 ° C gerührt. Nach dem Abkühlen wird der kristalline Feststoff abgesaugt und 15 Minuten mit Wasser verrührt

und erneut abgesaugt. Man erhält 39,4 g N-(4,6-Dichlor-pyrimidin-2-yl)-O-methyl-isoharnstoff (GC-Gehalt 98 %: 38,6 g; Ausbeute.: 69,9 % der Theorie), Schmelzpunkt 178 °C.

Beispiel (II-3)

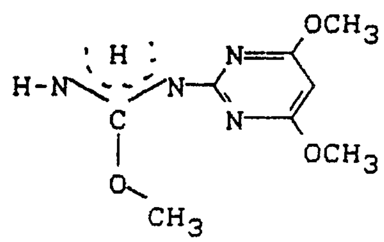

22,1 g (93,8 %ig: 0,0938 Mol) N-(4,6-Dichlor-pyrimidin-2-yl)-O-methyl-isoharnstoff werden in 200 ml Methanol suspendiert; dazu wird eine Lösung von 13,5 g (0,25 Mol) Natriummethylat in 100 ml Methanol tropfenweise gegeben. Das Reaktionsgemisch wird 16 Stunden unter Rückfluß erhitzt und dann eingeengt. Der Rückstand wird in Wasser aufgenommen und dreimal mit Methylenchlorid extrahiert. Von den vereinigten Extrakten wird das Lösungsmittel abdestilliert.
Man erhält 19,9 g (100 % der Theorie) N-(4,6-Dimethoxypyrimidin-2-yl)-O-methyl-isoharnstoff als kristallinen Rückstand vom Schmelzpunkt 98 °C.

Beispiel (II-4)

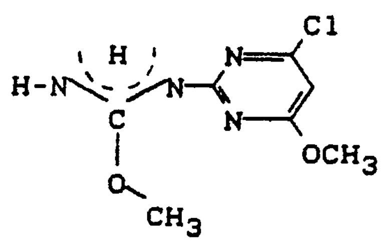

9,1 g (0,041 Mol) N-(4,6-Dichlor-pyrimidin-2-yl)-O-methyl-isoharnstoff werden in 80 ml Methanol vorgelegt. Bei 20 °C werden dann 2,2 g Natriummethylat (0,041 Mol) gelöst in 20 ml Methanol zugetropft. Man rührt 12 Stunden nach, engt ein, nimmt den Rückstand in Methylenchlorid auf, wäscht dreimal mit Wasser und engt erneut ein.
Man erhält 7,6 g (86 % der Theorie) N-(4-Chlor-6-methoxy-pyrimidin-2-yl)-O-methyl-isoharnstoff in Form weißer Kristalle vom Schmelzpunkt 118-120 °C.

Analog erhält man:

Beispiel (II-5)

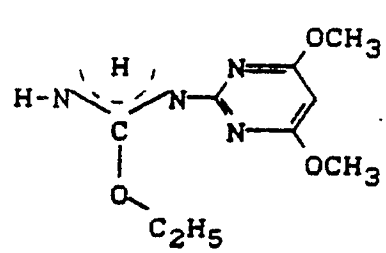

N-(4,6-Dimethoxy-pyrimidin-2-yl)-O-ethyl-isoharnstoff
Ausbeute: 72 % (der Theorie); Schmelzpunkt: 96 °C.

Beispiel (II-6)

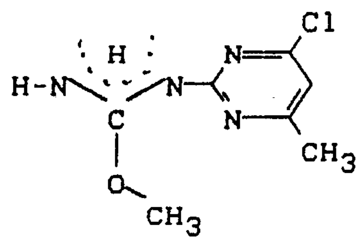

N-(4-Chlor-6-methyl-pyrimidin-2-yl)-O-methylisoharnstoff; Schmelzpunkt: 93 °C.

Beispiel (II-7)

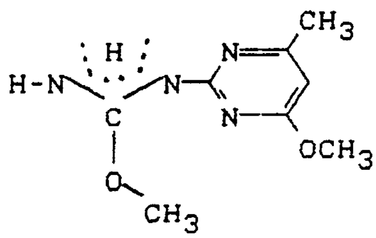

N-(4-Methoxy-6-methyl-pyrimidin-2-yl)-O-methyl-isoharnstoff; Schmelzpunkt: 114 °C.

Beispiel 2

Analog zu Beispiel 1, ebenfalls unter Einsatz von Dicyclohexylamin als Base, erhält man die folgende Verbindung:

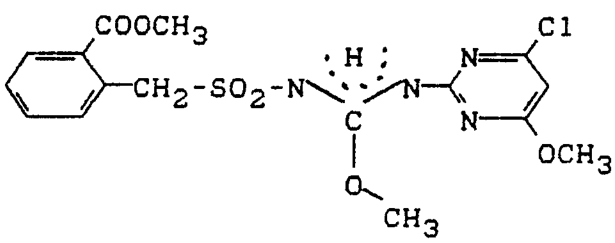

N-(4-Chlor-6-methoxy-pyrimidin-2-yl)-N′-(2-methoxycarbonyl-benzylsulfonyl)-O-methyl-isoharnstoff.
Ausbeute: 96 % der Theorie; Schmelzpunkt: 128-132 °C.

Beispiel 3

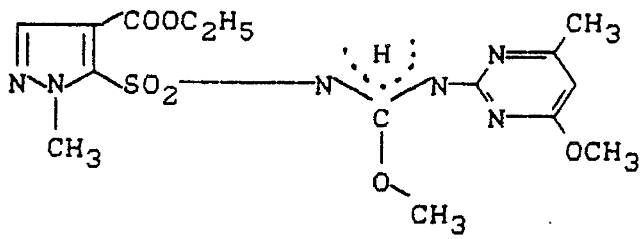

1,2 g (6,12 mMol) N-(4-Methyl-6-methoxy-pyrimidin-2-yl)-O-methyl-isoharnstoff und 0,9 g (6,7 mMol) N,N-Dimethylbenzylamin werden in 10 ml Dimethoxyethan vorgelegt und bei 5 - 10 °C werden 1,9 g (90 %ig, 6,7 mMol) (1-Methyl- 4-ethoxycarbonyl-pyrazol-5-yl)-sulfonsäurechlorid zugegeben. Man läßt auf Raumtemperatur kommen und rührt 10 Stunden nach. Man engt ein, löst den Rückstand in Methylenchlorid und wäscht diese Lösung dreimal mit Wasser. Nach Trocknen und Abdestillieren des Methylenchlorids erhält man 2,1 g gelbes Öl, welches zu 88 % (gemäß HPLC) aus dem gewünschten N-(4-Methoxy-6-methyl-pyrimidin-2-yl)-N′-(1-methyl-4-ethoxycarbonyl-pyrazol-5-yl)-O-methyl-isoharnstoff besteht: dies entspricht einer Ausbeute von 73 % der Theorie.

$^1$H-NMR ($CDCl_3$) δ: 1,3 ppm (Triplett, 3H);
2,45 ppm (s, 3H);
3,95 ppm (s, 3H);
3,975 ppm (s, 3H);
4,26 ppm (m, 3H + 2H);
6,3 ppm (s, 1H);
7,8 ppm (s, 1H);
1,7 ppm (breit, NH ).

**Ansprüche**

1. Verfahren zur Herstellung von Sulfonylisoharnstoffen der allgemeinen Formel (I),

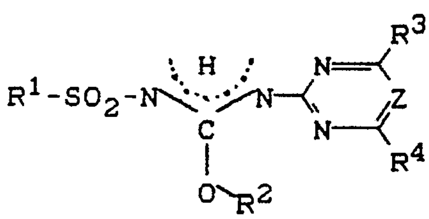

(I)

in welcher
$R^1$ für einen gegebenenfalls substituierten Rest aus der Reihe Alkyl, Aralkyl, Aryl oder Heteroaryl steht,
$R^2$ für Alkyl oder Aralkyl steht,
$R^3$ für Wasserstoff, Halogen oder für jeweils gegebenenfalls substituiertes Alkyl oder Alkoxy steht,
$R^4$ für Wasserstoff, Halogen oder für jeweils gegebenenfalls substituiertes Alkyl oder Alkoxy steht und
Z für Stickstoff oder eine CH-Gruppierung steht,
dadurch gekennzeichnet, daß man Isoharnstoffe der allgemeinen Formel (II),

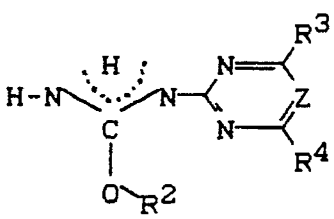

(II)

in welcher
$R^2$, $R^3$, $R^4$ und Z die oben angegebenen Bedeutungen haben,

mit Sulfonsäurehalogeniden der allgemeinen Formel III

$R^1$-$SO_2$-X (III)

in welcher

$R^1$ die oben angegebene Bedeutung hat und

X für Halogen steht,

in Gegenwart eines Säureakzeptors und gegebenenfalls in Gegenwart eines Verdünnungsmittels bei Temperaturen zwischen -20 °C und +50 °C umsetzt.

2. Verfahren zur Herstellung von Isoharnstoffen der Formel (II),

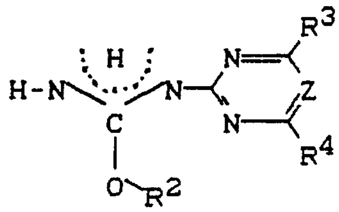

(II)

in welcher

$R^2$ für Alkyl oder Aralkyl steht,

$R^3$ für Wasserstoff, Halogen oder für jeweils gegebenenfalls substituiertes Alkyl oder Alkoxy steht,

$R^4$ für Wasserstoff, Halogen oder für jeweils gegebenenfalls substituiertes Alkyl oder Alkoxy steht und

Z für Stickstoff oder eine CH-Gruppierung steht,

dadurch gekennzeichnet, daß man Isoharnstoffe der Formel (IV),

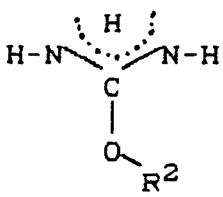

(IV)

in welcher

$R^2$ die oben angegebene Bedeutung hat,

- oder Säureaddukte von Verbindungen der Formel (IV) -

mit Azinen der allgemeinen Formel (V)

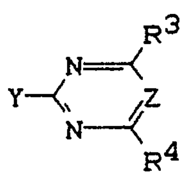

(V)

in welcher

$R^3$ und $R^4$ die oben angegebenen Bedeutungen haben,

Y für Halogen oder Alkylsulfonyl steht und

Z für Stickstoff oder eine CH-Gruppierung steht,

in Gegenwart eines Säureakzeptors und in Gegenwart eines Verdünnungsmittels bei Temperaturen zwischen 0 °C und 200 °C umsetzt.

3. Verfahren nach Anspruch 1, dadurch gekennzeichnet, daß man die als Zwischenprodukte benötigten Isoharnstoffe der Formel (II) mit Hilfe des Verfahrens gemäß Anspruch 2 herstellt.

4. Verfahren nach Anspruch 1, dadurch gekennzeichnet, daß man bei Temperaturen zwischen 0 °C und 30 °C arbeitet.

5. Verfahren nach Anspruch 2, dadurch gekennzeichnet, daß man bei Temperaturen zwischen 10 °C und 150 °C arbeitet.

6. Isoharnstoffe der Formel (IIa),

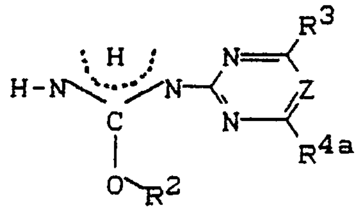

(IIa)

in welcher
$R^2$ für Alkyl oder Aralkyl steht,
$R^3$ für Wasserstoff, Halogen oder für jeweils gegebenenfalls substituiertes Alkyl oder Alkoxy steht,
$R^{4a}$ für Wasserstoff, Halogen oder für gegebenenfalls substituiertes Alkoxy steht und
Z für Stickstoff oder eine CH-Gruppierung steht.

7. Isoharnstoffe der Formel (IIa) gemäß Anspruch 6, dadurch gekennzeichnet, daß darin
$R^2$ für $C_1$-$C_4$-Alkyl oder für Benzyl steht,
$R^3$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy oder $C_1$-$C_2$-Alkoxy-$C_1$-$C_2$-alkoxy steht,
$R^{4a}$ für Wasserstoff, Fluor, Chlor, Brom, $C_1$-$C_4$-Alkoxy, $C_1$-$C_4$-Halogenalkoxy oder $C_1$-$C_2$-Alkoxy-$C_1$-$C_2$-alkoxy steht und
Z für Stickstoff oder eine CH-Gruppierung steht.

8. Isoharnstoffe der Formel (IIa) gemäß Anspruch 6, dadurch gekennzeichnet, daß darin
$R^2$ für Methyl oder Ethyl steht,
$R^3$ für Chlor, Methyl, Ethyl, Trifluormethyl, Methoxymethyl, Ethoxymethyl, Methoxy, Ethoxy oder Difluormethoxy steht,
$R^{4a}$ für Chlor, Methoxy, Ethoxy oder Difluormethoxy steht, und
Z für eine CH-Gruppierung steht.

9. Zweistufiges Verfahren zur Herstellung von N-(4,6-Dialkoxy-pyrimidin-2yl)-isoharnstoffen der Formel

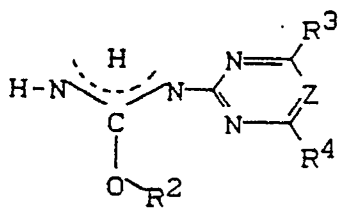

(II)

in welcher
Z für CH steht,
$R^2$ für Alkyl oder Aralkyl steht und
$R^3$ und $R^4$ jeweils für Alkoxy stehen,
dadurch gekennzeichnet, daß man

(1) 2,4,6-Trichlor-pyrimidin der Formel

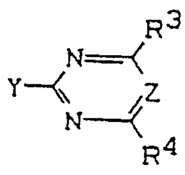

(V)

in welcher
Z für CH steht und
Y, $R^3$ und $R^4$ für Chlor stehen,
mit einem Isoharnstoff der Formel

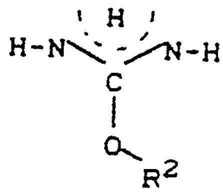

(IV)

in welcher
$R^2$ für Alkyl oder Aralkyl steht,
oder mit einem Säureaddukt hiervon in Gegenwart eines Säureakzeptors und in Gegenwart eines Verdünnungsmittels bei Temperaturen zwischen 0° C und 200° C umsetzt und anschließend
(2) die hierbei erhaltenen N-(4,6-Dichlorpyrimidin-2-yl)-isoharnstoffe der Formel

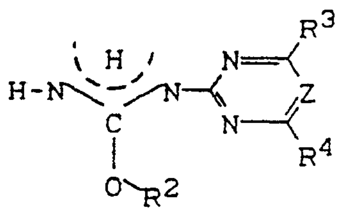

(II)

in welcher
Z für CH steht,
$R^2$ für Alkyl oder Aralkyl steht und
$R^3$ und $R^4$ für Chlor stehen,
mit Alkalimetallalkanolaten in Gegenwart der entsprechenden Alkohole bei Temperaturen zwischen 0° C und 150° C umsetzt.